# EUROPEAN PATENT SPECIFICATION

(11) **EP 1 708 679 B1**
(45) Date of publication and mention of the grant of the patent: **12.11.2008**
(21) Application number: 04798662.5
(22) Date of filing: 24.11.2004
(51) Int. Cl.: A61K 31/137, A61K 31/167, A61K 31/573, A61K 9/00

(54) **PHARMACEUTICAL SPRAY FORMULATION COMPRISING A HYDRO FLUOR ALKANE AND AN ACYLATED CYCLODEXTRIN**
PHARMAZEUTISCHE SPRAYFORMULIERUNG MIT EINEM HYDROFLUORALKAN UND EINEM ACYLIERTEN CYCLODEXTRIN
FORMULATION PHARMACEUTIQUE D'UN SPRAY COMPRENANT UN HYDROFLUOROALCANE ET UNE CYCLODEXTRINE ACYLEE

(30) Priority: 26.11.2003 SE 0303179
(43) Date of publication of application: 11.10.2006
(73) Proprietor: AstraZeneca AB, 151 85 Södertälje (SE)
(72) Inventor: ROGUEDA, Philippe, Loughborough, Leicestershire LE11 5RH (GB)
(74) Representative: Summersell, Richard John
(86) International application number: PCT/GB2004/004957
(87) International publication number: WO 2005/053637

(56) References cited:
- DE-A1- 10 205 087
- WILLIAMS III R O ET AL: "Influence of formulation technique for hydroxypropyl-beta-cyclodextrin on the stability of aspirin in HFA 134a" EUROPEAN JOURNAL OF PHARMACEUTICS AND BIOPHARMACEUTICS, ELSEVIER SCIENCE PUBLISHERS B.V., AMSTERDAM, NL, vol. 47, no. 2, 1 March 1999 (1999-03-01), pages 145-152, XP004257055 ISSN: 0939-6411
- STECKEL H ET AL: "A novel formulation technique for metered dose inhaler (MDI) suspensions" INTERNATIONAL JOURNAL OF PHARMACEUTICS, AMSTERDAM, NL, vol. 284, no. 1-2, 13 October 2004 (2004-10-13), pages 75-82, XP004580739 ISSN: 0378-5173

## Description

### Introduction

One of the modes of inhalation delivery of pharmaceutical substances is with pMDIs (pressure metered dose inhalers). These consist of a drug suspension or solution in a liquefied propellant, nowadays an HFA (hydro fluoro alkanes) or mixture of HFAs, such as HFA 227 or HFA 134a.

To form stable pMDI formulation it is often necessary to add stabilisers or solubilisers. The stabilisers can be polymers or surfactants, and help to reduce particle aggregation and phase separation in drug suspensions. The solubilisers can be organic solvents miscible with HFAs such as ethanol, and help to solubilise the drug in the HFAs. In many cases, the addition of both stabiliser and solubiliser may be necessary, when the stabilisers need a co-solvent for solubilisation for instance.

The range of excipients (stabilisers and solubilisers) that can be used to formulate HFA pMDIs is limited because of the poor solvent properties of the HFA propellants. As a consequence most of the patented inventions related to HFA pMDI formulations rely on the addition of both solubilisers and stabilisers. In this work however, excipients have been found which are naturally soluble in the HFAs in quantities large enough for them to be efficient suspension stabilisers, or to be used as solubilising agents.

Polymeric and surfactant stabilisers impart solubility to drug suspension in HFA by absorbing to the surface of the drug particles, and thus triggering a mechanism of steric stabilisation. For the stabilisers to be efficient, they need to be soluble in the dispersing medium to a suitable level, at least in excess of 0.5 %w/w, although this limit is excipient dependent.

The mode of action of solubilisers is simpler, as these act as solvents for the drug substance. As long as they are miscible with the HFAs, drugs can be brought into solution in the HFA-solubiliser mix. In the case of cyclodextrins, their mode of action is different. Cyclodextrins are able to form complexes with the drug molecules, and it is this complex that is solubilised in the HFAs.

### Background to the invention

Cyclodextrins have been used extensively for the formulation of pharmaceutical dosage forms, in particular to increase the solubility of otherwise poorly soluble drugs, or to impart controlled release properties.

More rarely have cyclodextrins been used for the formulation of inhalation products in HFAs. DE10205087 describes a metered dose inhaler composition comprising an active ingredient, a propellant, a modified cyclodextrin and a hydrophilic additive. Williams and Liu (European Journal of Pharmaceutics and Biopharmaceutics. 1999. 47. 145-152) describe a metered dose formulation which comprises HFA 134a, aspirin and hydroxypropyl beta cyclodextrin. Whereas Steckel and Wehle (International Journal of Pharmaceutics. 2004. 284. 75-82) describe a metered dose formulation which comprises an HFA propellant, budesonide and modified cyclodextrins.

Use of cyclodextrins in HFA formulations is rare because most cyclodextrins, in particular natural cyclodextrins, are insoluble in HFAs. In WO03/066031, cyclodextrins are used to stabilise drug suspensions. This however is only possible by the addition of at least one co-solvent (one hydrophilic additive, such as PEG; poly ethylene glycol), but preferably two (one hydrophilic additive and ethanol). These co-solvents solubilise the cyclodextrins, and thus make them useful as stabilisers.

The present invention has identified the correct cyclodextrins that do not require any co-solvents, and thus constitutes a major improvement to the invention of WO03/066031.

### Description of the Invention

It has now been found that some modified cyclodextrins are naturally soluble in HFA propellants. These are partially or fully acylated alpha (α), beta (β) or gamma (γ) cyclodextrins.

It has been found that the solubilised modified cyclodextrins were very good stabilisers for drug suspensions, and that due to the ability of cyclodextrin to act as complexing agents for drug molecules, they can also be used to form solution pMDIs.

In a first aspect the invention provides HFA formulations comprising a partially or fully acylated alpha (α), beta (β) or gamma (γ) cyclodextrin.

In one embodiment of the invention the formulations are in the form of a suspension.

In preferred embodiments, the invention provides stable dispersions for the pulmonary or nasal delivery of one or more active molecule, for local or systemic administration. It comprises a physical mixture of modified cyclodextrin with one or more active ingredients in a propellant or propellant mixture.

In a further aspect the invention provides a method for making a suspension formulation wherein modified cyclodextrins are first solubilised in HFA 134 or HFA 227, or a mixture of both, to the required concentration levels, from 0.001 %w/w to 20 %w/w, preferably from 0.001 %w/w to 10 % w/w, most preferably from 0.001 %w/w to 5 % w/w. The active ingredient (drug) is then added to the cyclodextrin-HFA solution and filled into a pMDI canister. Alternatively, known amounts of drug can be added to individual canisters and the cyclodextrin-HFA solution added to the known weight cans. The drug suspension thus formed can be homogenised by appropriate means: stirrer, ultrasonic energy etc.

In another aspect the invention concerns the formation of pMDI solution formulations. The drug and the cyclodextrin are first solubilised in a common solvent. The solution is allowed to equilibrate over a couple of days. When the complex is formed, it is extracted by, for example spray drying, freeze drying or supercritical fluid extraction. The solid formed is then solubilised in the HFA, or mixture thereof by virtue of the solubility of the modified cyclodextrin.

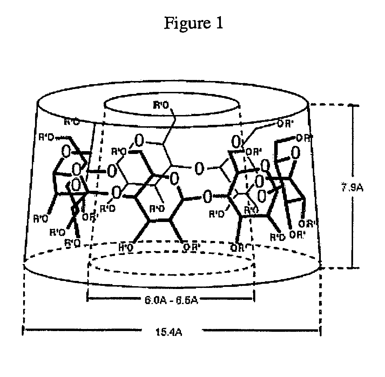

The modified cyclodextrin is a partially or fully substituted alpha (α), beta (β) or gamma (γ) cyclodextrin as shown on Figure 1, with R' an acryl group of the general formula; R-CO-. In particular the substituting group can be taken from the following selection: Acetyl, Acryloyl, Alanyl, Aminocarbonyl, β-Alanyl, aklyl Azelaoyl, Benzoyl, tert-Butoxy, Butynyl, Caproyl, Crotomoyl, Formyl, alkyl Glutaryl, Glycoloyl, Glycyl, Glyoxyloyl, Heptadecanoyl, Hydroperoxy, Hydroxyamino, Isobutynyl, Isovalenyl, Lactoyl, Lenyl, Levulinoyl, alkyl Malonyl, Mandeloyl, Methacryloyl, Myristoyl, Monanoyl, alkyl Oxalyl, Palmitoyl, alkyl Pimeloyl, Pivaloyl, Propanoyl, Salicyloyl, Seryl, Sorboyl, Stearoyl, alkyl Suberoyl, alkyl Succinyl, Theronyl, Tolnoyl, Valeryl, Valyl. Preferably the acetyl group is: Formyl, Acetyl, and Propanoyl.

The drug is any pharmaceuticaly active ingredient used in inhalation delivery. It can be micronised if needed for targeted delivery, such as in the treatment of respiratory diseases. It may be selected from any therapeutic or diagnostic agent. For example it may be from the group of antiallergics, bronchodilators, bronchoconstrictors, pulmonary lung surfactants, analgesics, antibiotics leukotrine inhibitors or antagonists, anticholinergics, mast cell inhibitors, antihistamines, antiinflammatories, antineoplastics, anaesthetics, anti-tuberculars, imaging agents, cardiovascular agents, enzymes, steroids, genetic material, viral vectors, antisense agents, proteins, peptides and combinations thereof.

In particular, the pharmacologically active agents in accordance with the present invention include glucocorticosteroids such as: budesonide, fluticasone (e.g. as propionate ester), mometasone (e.g. as furoate ester), beclomethasone (e.g. as 17-propionate or 17,21-dipropionate esters), ciclesonide, triamcinolone (e.g. as acetonide), flunisolide, zoticasone, flumoxonide, rofleponide, butixocort (e.g. as propionate ester), prednisolone, prednisone, tipredane, steroid esters according to WO 2002/12265, WO 2002/12266 and WO 2002/88167 (I) e.g. 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester and 6α,9α--difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, steroid esters according to DE 4129535 (II) and the like. Long-acting β₂ agonists, without limitation, include: salmeterol, formoterol, bambuterol, TA 2005 (chemically identified as 2(1H)-Quinolone, 8-hydroxy-5-[1-hydroxy-2-[[2-(4-methoxy-phenyl)-1-methylethyl]amino]ethyl]-monohydrochloride, [R-(R*,R*)] also identified by Chemical Abstract Service Registry Number 137888-11-0 and disclosed in U.S. Patent No 4.579.854, formanilide derivatives (III) e.g. 3-(4-{[6-({(2R)-2-[3-(formylamino)-4-hydroxyphenyl]-2-hydroxyethyl}amino)hexyl]oxy}-butyl)benzenesulfonamide as disclosed in WO 2002/76933, benzenesulfonamide derivatives (IV) e.g. 3-(4-{[6-({(2R)-2-hydroxy-2-[4-hydroxy-3-(hydroxymethyl)phenyl]ethylamino)-hexyl]oxy}butyl)benzenesulfonamide as disclosed in WO 2002/88167, hyaluronic acid and the like. Several of these compounds could be administered in the form of pharmacologically acceptable esters, salts, solvates, such as hydrates, or solvates of such esters or salts, if any. Both racemic mixtures as well as one or more optical isomers of the above compounds are within the scope of the invention.

The preferred pharmacologically active glucocorticosteroid agents for use in accordance with the present invention include mometasone furcate, ciclesonide, zoticasone, flumoxonide, steroids from WO 2002/88167 e.g. 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester and 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, steroids from DE 4129535, fluticasone propionate and budesonide, and even more preferred is budesonide. The preferred pharmacologically active long-acting β₂-agonist is salmeterol xinafoate, formanilide derivatives (III), benzenesulfonamide derivatives (IV) and formoterol (e.g. as fumarate dihydrate) and even more preferred is formoterol fumarate dihydrate.

Combinations of pharmacologicaly active ingredients include fluticasone propionate/salmeterol xinafoate, ciclesonide/formoterol fumarate dihydrate, mometasone furoate/formoterol fumarate dihydrate, fluticasone propionate/formoterol fumarate dihydrate, and budesonide/formoterol fumarate dihydrate.

Other preferred combinations include steroids from WO 2002/88167 /formanilide derivatives from WO 2002/76933, steroids from WO 2002/88167/benzenesulfonamide derivatives from WO 2002/88167, steroids from DE 4129535/formoterol fumarate dihydrate, zoteasone/benzenesulfonamide derivatives from WO 2002/88167 and zoticasone/formanilide derivative.

A most preferred combination is budesonide/formoterol fumarate dihydrate.

Figure 1 shows the general structure of a modified β cylcodextrin. " R' " are the groups that can be substituted on the cyclodextrin ring with acyl functions.

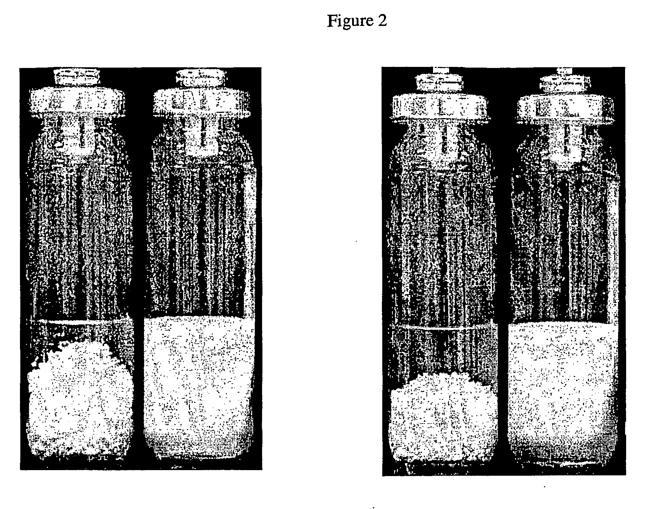

Figure 2 shows pMDI suspensions of terbutaline sulphate in HFA 227, with (right can) and without (left can) peracetylated β cyclodextrin. Left sample: terbutaline sulphate (C= 1.03 %w/w) in HFA 227, 10 sec after shaking. Right sample: terbutaline sulphate (C= 0.97 %w/w) in saturated solution of in HFA 227, 5 min 23 sec after shaking

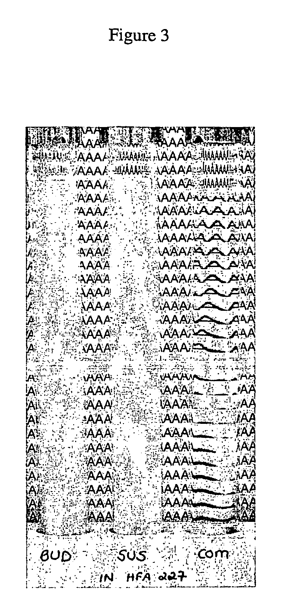

Figure 3 shows an HFA pMDI solution formulation in HFA 227. The left tube contains budesonide in HFA 227 at C= 0.04 %w/w. The tube in the middle contains a physical mixture of budesonide (C= 0.04 %w/w) and peracetylated β cyclodextrin (C= 0.04 %w/w) as prepared in the suspension section. The right tube contains the spray dried 1:1 complex of budesonide (C= 0.04 %w/w) and peracetylated β cycloxdextrin (C= 0.04 %w/w).

### EXAMPLES

### Solubility of peracetylated cyclodextrins in HFAs

The solubility of peracetylated cyclodextrins (alpha, beta and gamma) in HFA propellants was assessed visually in clear PET vials crimped with a continuous pMDI valve. Known amounts of cyclodextrins were weighed into the PET vials. Continuous valves were crimped on the vial, and HFAs were added under pressure to the required amount. The samples were left to equilibrate over one week and were visually inspected. The solubility of the modified cyclodextrin at room temperature was evaluated by discriminating between samples that were clear by opposition to those samples that still had solid particles in suspension.

Following this method, the solubility of peracetylated alpha (α) cyclodextrin in HFA 227 was shown to be above 0.5 %w/w. Its solubility in HFA 134a was shown to be above 1 %w/w.

The solubility of peracetylated beta (β) cyclodextrin in HFA 227 was shown to be above 0.1 %w/w. Its solubility in HFA 134a was shown to be above 1 %w/w.

The solubility of peracetylated gamma (γ) cyclodextrin in HFA 227 was shown to be above 1 %w/w. Its solubility in HFA 134a was shown to be above 1 %w/w.
For reference the corresponding natural cyclodextrins are insoluble in both propellants. Peracetylated beta (β) cyclodextrin was chosen to exemplify the invention.

### Efficacy of peracetylated beta (β) cyclodextrin as a suspension stabiliser

The efficacy of peracetylated beta (β) cyclodextrin as an HFA drug suspension stabiliser was tested with a selection of drug compounds.
First, reference samples of pure drugs in HFAs were prepared. Then samples with solubilised peracetylated beta (β) cyclodextrin and micronised drug were prepared. The concentration of drug in these samples was the same as in the reference samples. The cyclodextrin concentration was at saturation level.

The reference samples (pure drug) were prepared by weighing the drug in a PET vial. A valve was then crimped on the vial and propellant added under pressure. The vial was then sonicated for at least 15 minutes and left to equilibrate for one week.

The drug - cyclodextrin samples were prepared by weighing a known amount of drug in a PET vial. The vial was then crimped with a continuous valve. The solution of peracetylated beta (β) cyclodextrin was prepared thus: a large amount of cyclodextrin was placed in large metallic can, it was crimped and filled with one of the propellants. This can was left to equilibrate for at least a week at room temperature. When equilibration was reached, the saturated cyclodextrin solution was filtered through a 0.2 nm PTFE filter into the PET vials containing the known drug amounts. This way the maximum amount of soluble cyclodextrin was used in the suspensions. The concentration of drugs in the vials was then calculated through accurate recording of the weights of propellant/cyclodextrin added.

The phase separation phenomenon present in both samples (pure drug and with the cyclodextrin) was then recorded photographically.

A selection of HFA pMDI suspension examples follows.

### TERBUTALINE SULPHATE

### Samples in HFA 227

2 samples were prepared: a reference sample containing pure drug at C= 1.03 %w/w, and a sample with a similar drug concentration (C= 0.97 %w/w) with peracetylated β cyclodextrin at its saturation level. The samples were prepared by weight with HFA 227 added by pressure filling. The sample containing the modified cyclodextrin was far more stable than the sample without cyclodextrin. Its phase separation, flocculation and sedimentation, was much slower, the suspension was much finer, and head space adhesion was less than for the pure drug sample. See Figure 2.

### Samples in HFA 134a

A method similar to the one in the previous example was used to prepare a reference sample containing pure drug (C= 1.16 %w/w), and a sample containing the drug (C=1.18 %w/w) and the modified cyclodextrin at its saturated level. The quality of the stabilised drug suspension was much improved in a similar way as in the previous example upon addition of modified cyclodextrin, i.e. its phase separation, flocculation and sedimentation, was much slower, the suspension was much finer, and head space adhesion was less than for the pure drug sample.

### SALBUTAMOL BASE

### Samples in HFA 227

A method similar to the one in the previous example was used to prepare a reference sample containing pure drug (C= 0.1 %w/w), and a sample containing the drug (C=0.11 %w/w) and the modified cyclodextrin at its saturated level. The quality of the stabilised drug suspension was improved in a similar way as in the previous example upon addition of modified cyclodextrin, i.e. its phase separation, flocculation and sedimentation, was much slower, the suspension was much finer, and head space adhesion was less than for the pure drug sample.

### Samples in HFA 134a

A method similar to the one in the previous example was used to prepare a reference sample containing pure drug (C= 0.13 %w/w), and a sample containing the drug (C=0.11 %w/w) and the modified cyclodextrin at its saturated level. The quality of the stabilised drug suspension was improved in a similar way as in the previous example upon addition of modified cyclodextrin, i.e. its phase separation, flocculation and sedimentation, was much slower, the suspension was much finer, and head space adhesion was less than for the pure drug sample.

### SALBUTAMOL SULPHATE

### Samples in HFA 227

A method similar to the one in the previous example was used to prepare a reference sample containing pure drug (C=0.11 %w/w), and a sample containing the drug (C= 0.12 %w/w) and the modified cyclodextrin at its saturated level. The quality of the stabilised drug suspension was improved in a similar way as in the previous example upon addition of modified cyclodextrin, i.e. its phase separation, flocculation and sedimentation, was much slower, the suspension was much finer, and head space adhesion was less than for the pure drug sample.

### Samples in HFA 134a

A method similar to the one in the previous example was used to prepare a reference sample containing pure drug (C= 0.14 %w/w), and a sample containing the drug (C= 0.11 %w/w) and the modified cyclodextrin at its saturated level. The quality of the stabilised drug suspension was improved in a similar way as in the previous example upon addition of modified cyclodextrin, i.e. its phase separation, flocculation and sedimentation, was much slower, the suspension was much finer, and head space adhesion was less than for the pure drug sample.

### FORMOTEROL FUMARATE DIHYDRATE

### Samples in HFA 227

A method similar to the one in the previous example was used to prepare a reference sample containing pure drug (C=0.017 %w/w), and a sample containing the drug (C= 0.0014 %w/w) and the modified cyclodextrin at its saturated level. The quality of the stabilised drug suspension was improved in a similar way as in the previous example upon addition of modified cyclodextrin, i.e its phase separation, flocculation and sedimentation, was much slower, the suspension was much finer, and head space adhesion was less than for the pure drug sample.

### Samples in HFA 134a

A method similar to the one in the previous example was used to prepare a reference sample containing pure drug (C= 0.021 %w/w), and a sample containing the drug (C= 0.019 %w/w) and the modified cyclodextrin at its saturated level. The quality of the stabilised drug suspension was improved in a similar way as in the previous example upon addition of modified cyclodextrin, i.e. its phase separation, flocculation and sedimentation, was much slower, the suspension was much finer, and head space adhesion was less than for the pure drug sample.

### BUDESONIDE

### Samples in HFA 227

A method similar to the one in the previous example was used to prepare a reference sample containing pure drug (C= 0.27 %w/w), and a sample containing the drug (C= 0.27 %w/w) and the modified cyclodextrin at its saturated level. The quality of the stabilised drug suspension was much improved upon addition of the modified cyclodextrin.

### Samples in HFA 134a

A method similar to the one in the previous example was used to prepare a reference sample containing pure drug (C= 0.32 %w/w), and a sample containing the drug (C= 0.25 %w/w) and the modified cyclodextrin at its saturated level. The quality of the stabilised drug suspension was improved in a similar way as in the previous example upon addition of modified cyclodextrin, i.e its phase separation, flocculation and sedimentation, was much slower, the suspension was much finer, and head space adhesion was less than for the pure drug sample.

The following Examples all relate to the formation of HFA pMDI solution formulations.

### Efficacy of peracetylated β cyclodextrin as a solubiliser

The efficacy of the modified cyclodextrin as a solubiliser was tested with budesonide and peracetylated β cyclodextrin. A good solvent for these two molecules is chloroform. Five solutions of budesonide with cyclodextrin in chloroform were prepared by weight. They were prepared so as to have an increasing molar ratio of cyclodextrin vs. budesonide, i.e. the following budesonide: cyclodextrin ratios were prepared: 1:1, 1:2, 1:3, 1:4 and 1:5. These solutions were left to equilibrate for a couple of days. The solutions were then spray dried to form solid particles of drug cyclodextrin complex. The dry powders were then weighed in glass tubes and known amount of propellant added under pressure. Visual observations were performed. For the sake of comparison, 2 types of reference samples were prepared. The first reference sample was a suspension of pure drug at the same concentration as the drug in the drug-cyclodextrin spraydried complex. The second reference sample was a physical mixture of drug and cyclodextrin at the same concentrations as in the spray dried complex.

### 1:1 budesonide-cyclodextrin complex in HFA 227

A 1:1 fixed molar ratio budesonide: peracetylated β cyclodextrin solution was prepared in chloroform. This solution was spray dried. The spray dried powder dissolved in HFA 227, upon addition of the propellant. The dissolution was instantaneous and led to a clear solution.

Figure 3 shows the photographic evidence of the formation of an HFA pMDI solution formulation with a drug-cyclodextrin complex. The tube on the left contains budesonide in HFA 227 at C= 0.04 %w/w, the tube in the middle contains a physical mixture of budesonide (C= 0.04 %w/w) and peracetylated β cyclodextrin (C= 0.04 %w/w) as prepared in the previous section. These 2 samples are suspensions. The 3^{rd} tube, on the right, contains the spray dried complex of budesonide (C= 0.04 %w/w) and peracetylated β cycloxdextrin (C= 0.04 %w/w). This 3^{rd} tube contains a clear solution.

It is therefore possible to form an HFA pMDI solution formulation with modified cyclodextrins.

### 1:2 budesonide-cyclodextrin complex in HFA 227

A 1:2 fixed molar ratio budesonide: peracetylated β cyclodextrin solution was prepared in chloroform. This solution was spray dried. The spray dried powder dissolved in HFA 227, upon addition of the propellant. The dissolution was instantaneous.

### 1:3 budesonide-cyclodextrin complex in HFA 227

A 1:3 fixed molar ratio budesonide: peracetylated β cyclodextrin solution was prepared in chloroform. This solution was spray dried. The spray dried powder dissolved in HFA 227, upon addition of the propellant. The dissolution was instantaneous.

### 1:4 budesonide-cyclodextrin complex in HFA 227

A 1:4 fixed molar ratio budesonide: peracetylated β cyclodextrin solution was prepared in chloroform. This solution was spray dried. The spray dried powder dissolved in HFA 227, upon addition of the propellant. The dissolution was instantaneous.

### 1:5 budesonide-cylcodextrin complex in HFA 227

A 1:5 fixed molar ratio budesonide: peracetylated β cyclodextrin solution was prepared in chloroform. This solution was spray dried. The spray dried powder dissolved in HFA 227, upon addition of the propellant. The dissolution was instantaneous.

### Conclusions

Figure 2 shows that the addition of an acylated β cyclodextrin to an HFA pMDI drug suspension improves the suspension properties dramatically. In particular it helps to form a finely dispersed suspensions, showing little to no device adhesion. These suspensions phase separate at a much lower rate than cyclodextrin fee suspensions, and are easily re-dispersible. Further more no sign of agglomeration is visible on storage.

Figure 3 shows that it is possible to develop an HFA pMDI solution formulation by appropriate treatment of the drug-cyclodextrin complex.

## Claims

1. An HFA drug formulation comprising a partially or fully acylated alpha (α), beta (β) or gamma (γ) cyclodextrin.

2. A formulation according to claim 1 in which the HFA is HFA 134a, 227 or a mixture thereof.

3. A formulation according to claim 1 or 2 in the form of a solution.

4. A formulation according to claim 1 or 2 in the form of a suspension.

5. A formulation according to any one of clams 1 to 4 in which the cyclodextrin is acylated with one or more groups selected from Acetyl, Acryloyl, Alanyl, Aminocarbonyl, β-Alanyl, aklyl Azelaoyl, Benzoyl, tert-Butoxy, Butynyl, Caproyl, Crotomoyl, Formyl, alkyl Glutaryl, Glycoloyl, Glycyl, Glyoxyloyl, Heptadecanoyl, Hydroperoxy, Hydroxyamino, Isobutynyl, Isovalenyl, Lactoyl, Lenyl, Levulinoyl, alkyl Malonyl, Mandeloyl, Methacryloyl, Myristoyl, Monanoyl, alkyl Oxalyl, Palmitoyl, alkyl Pimeloyl, Pivaloyl, Propanoyl, Salicyloyl, Seryl, Sorboyl, Stearoyl, alkyl Suberoyl, alkyl Succinyl, Theronyl, Tolnoyl, Valeryl or Valyl.

6. A formulation according to any one of claims 1 to 5 in which the drug is fluticasone propionate, beclomethasone dipropionate, flunisolide, budesonide, tipredane, cortisone, prednisone, ephedrine, adrenaline, fenoterol, formoterol, isoprenaline, metaproterenol, sabutamol, albuterol, salmeterol, terbutaline and pharmaceutically acceptable salts and solvates thereof and combinations thereof.

7. A formulation according to any one of claims 1 to 6 in which there are two drugs and these are budesonide and formoterol fumarate dihydrate.

8. A formulation according to any one of claims 1 to 7 for the treatment or prophylaxis of a respiratory disease.

9. A formulation according to any one of claims 1 to 7 for the treatment or prophylaxis of asthma or COPD.

10. A pressure metered dose inhaler comprising an HFA drug formulation comprising a partially or fully acylated alpha (α), beta (β) or gamma (γ) cyclodextrin in the form of a solution.

## Patentansprüche

1. HFA-Arzneimittelformulierung, enthaltend teilweise oder vollständig acyliertes Alpha-(α-), Beta-(β-) oder Gamma-(γ-) Cyclodextrin.

2. Formulierung nach Anspruch 1, wobei es sich bei dem HFA um HFA 134a, 227 oder eine Mischung davon handelt.

3. Formulierung nach Anspruch 1 oder 2 in Form einer Lösung.

4. Formulierung nach Anspruch 1 oder 2 in Form einer Suspension.

5. Formulierung nach einem der Ansprüche 1 bis 4, wobei das Cyclodextrin durch eine oder mehrere Gruppen ausgewählt aus Acetyl, Acryloyl, Alanyl, Aminocarbonyl, β-Alanyl, Alkylazelaoyl, Benzoyl, tert.-Butoxy, Butinyl, Caproyl, Crotomoyl, Formyl, Alkylglutaryl, Glycoloyl, Glycyl, Glyoxyloyl, Heptadecanoyl, Hydroperoxy, Hydroxyamino, Isobutynyl, Isovalenyl, Lactoyl, Lenyl, Levulinoyl, Alkylmalonyl, Mandeloyl, Methacryloyl, Myristoyl, Monanoyl, Alkyloxalyl, Palmitoyl, Alkylpimeloyl, Pivaloyl, Propanoyl, Salicyloyl, Seryl, Sorboyl, Stearoyl, Alkylsuberoyl, Alkylsuccinyl, Theronyl, Tolnoyl, Valeryl oder Valyl acyliert ist.

6. Formulierung nach einem der Ansprüche 1 bis 5, wobei es sich bei dem Arzneimittel um Fluticasonpropionat, Beclomethasondipropionat, Flunisolid, Budesonid, Tipredan, Cortison, Prednison, Ephedrin, Adrenalin, Fenoterol, Formoterol, Isoprenalin, Metaproterenol, Sabutamol, Albuterol, Salmeterol, Terbutalin oder ein pharmazeutisch annehmbares Salz oder Solvat davon oder eine Kombination davon handelt.

7. Formulierung nach einem der Ansprüche 1 bis 6, bei der zwei Arzneimittel vorliegen, bei denen es sich um Budesonid und Formoterolfumarat-Dihydrat handelt.

8. Formulierung nach einem der Ansprüche 1 bis 7 zur Behandlung oder Prophylaxe einer Atemwegserkrankung.

9. Formulierung nach einem der Ansprüche 1 bis 7 zur Behandlung oder Prophylaxe von Asthma oder COPD.

10. Unter Druck stehendes Dosierinhalationsgerät, enthaltend eine HFA-Arzneimittelformulierung enthaltend ein teilweise oder vollständig acyliertes Alpha-(α-), Beta-(β-) oder Gamma-(γ-) Cyclodextrin in Form einer Lösung.

## Revendications

1. Formulation médicamenteuse d'HFA comprenant une cyclodextrine alpha (α), bêta (β) ou gamma (γ) partiellement ou totalement acylée.

2. Formulation selon la revendication 1, **caractérisée en ce que** l'HFA est l'HFA 134a, 227 ou un mélange de ceux-ci.

3. Formulation selon la revendication 1 ou 2, sous forme d'une solution.

4. Formulation selon la revendication 1 ou 2, sous forme d'une suspension.

5. Formulation selon l'une quelconque des revendications 1 à 4, dans laquelle la cyclodextrine est acylée avec un ou plusieurs groupements choisis parmi Acétyle, Acryloyle, Alanyle, Aminocarbonyle, β-Alanyle, alkyl Azélaoyle, Benzoyle, tert-Butoxy, Butynyle, Caproyle, Crotomoyle, Formyle, alkyl Glutaryle, Glycoloyle, Glycyle, Glyoxyloyle, Heptadécanoyle, Hydroperoxy, Hydroxyamino, Isobutynyle, Isovalényle, Lactoyle, Lényle, Lévulinoyle, alkyl Malonyle, Mandeloyle, Méthacryloyle, Myristoyle, Monanoyle, alkyl Oxalyle, Palmitoyle, alkyl Pimeloyle, Pivaloyle, Propanoyle, Salicyloyle, Séryle, Sorboyle, Stéaroyle, alkyl Subéroyle, alkyl Succinyle, Théronyle, Tolnoyle, Valéryle ou Valyle.

6. Formulation selon l'une quelconque des revendications 1 à 5, **caractérisée en ce que** le médicament est le propionate de fluticasone, le dipropionate de béclométhasone, le flunisolide, le budésonide, le tiprédane, la cortisone, la prédnisone, l'éphédrine, l'adrélanine, le fénotérol, le formotérol, l'isoprénaline, le métaprotérénol, le sabutamol, l'albutérol, le salmétérol, la terbutaline et les sels et les solvates pharmaceutiquement acceptables de ceux-ci et les combinaisons de ceux-ci.

7. Formulation selon l'une quelconque des revendications 1 à 6, dans laquelle il y a deux médicaments et il s'agit de budésonide et de fumarate de formotérol dihydraté.

8. Formulation selon l'une quelconque des revendications 1 à 7, destinée au traitement ou à la prophylaxie d'une maladie respiratoire.

9. Formulation selon l'une quelconque des revendications 1 à 7, destinée au traitement ou à la prophylaxie de l'asthme ou de la BPCO.

10. Inhalateur doseur sous pression comprenant une formulation médicamenteuse d'HFA comprenant une cyclodextrine alpha (α), bêta (β) ou gamma (γ) partiellement ou totalement acylée sous forme d'une solution.
